(19)

**Europäisches Patentamt**
**European Patent Office**
**Office européen des brevets**

(11) **EP 4 742 253 A1**

(12) **EUROPEAN PATENT APPLICATION**

(43) Date of publication:
**13.05.2026 Bulletin 2026/20**

(21) Application number: **24212149.9**

(22) Date of filing: **11.11.2024**

(51) International Patent Classification (IPC):
**G16C 60/00** (2019.01)   **G01N 33/49** (2006.01)
**G16B 5/30** (2019.01)

(52) Cooperative Patent Classification (CPC):
**G16B 5/30; G01N 33/4925; G16C 20/10;**
**G16C 20/20**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Aalborg Universitet**
**9220 Aalborg Øst (DK)**

(72) Inventors:
• **Rees, Stephen Edward**
**9260 Gistrup (DK)**
• **Thomsen, Lars Pilegaard**
**9520 Skørping (DK)**
• **Shastri, Lisha**
**2500 Valby (DK)**
• **Nevirian, Bahareh**
**9220 Aalborg Øst (DK)**

(74) Representative: **Plougmann Vingtoft a/s**
**Strandvejen 70**
**2900 Hellerup (DK)**

(54) **A METHOD FOR COMPENSATING THE EFFECTS OF GAS CONTAMINATION FOR A VENOUS BLOOD SAMPLE**

(57)    The invention relates to a computer-implemented method for compensating the effects of gas contamination of acid-base, oxygenation and/or electrolyte status for a venous blood sample, or blood sampled elsewhere, taken in a vacuum tube from a subject, such as human patient. The vacuum tube has a volume of residual gas ($V_g$) together with the blood. The method has a mathematical model with physiological sub-models simulating development in blood together with this residual gas, including sub-models describing: 1) the diffusion of gasses between the residual gas and the blood sample, 2) the effects of the diffusing gas in the blood in the vacuum tube, and 3) the effects of the diffusing gas in the residual gas in the vacuum tube. The mathematical model then calculates a modified set of blood variables (BV) to compensate for the physical and/or chemical interactions between the residual gasses and the blood sample in the time following sampling and before blood gas measurement. By accounting for this contamination of the blood sample, calculation of the actual values of blood variables seen in the patient is possible.

FIG. 2

Processed by Luminess, 75001 PARIS (FR)

EP 4 742 253 A1

**Description**

FIELD OF THE INVENTION

The present invention relates to a computer-implemented method for compensating the effects of gas or air contamination of acid-base, oxygenation and/or electrolyte status for a venous blood sample, or blood sampled elsewhere, taken in a vacuum based blood collection tube (hereafter referred to as a vacuum tube). The invention also relates to a corresponding system for measurement of blood, and a corresponding computer programme product.

BACKGROUND OF THE INVENTION

Measurement of blood acid-base status is usually performed in arterial or venous blood samples using standardized blood gas sampling tubes. In contrast, collection of blood for other purposes, often venous blood, are usually sampled in vacuum tubes.

Vacuum tubes function such that the under-pressure of the tube draws blood from the patient. This is simple, and therefore beneficial but means that, following the sampling, the tube is partially filled with air. The volume of the air depends on the design of the tube and hence the initial under-pressure, with volumes between one-quarter to three-quarters of the tube not uncommon.

The contamination of the blood sample with the remaining volume of air means that values of blood gasses will be contaminated resulting in significant bias in measurement of acid-base status. This prevents the routine use of vacuum tubes for all venous samples which could jeopardize standardized sampling and improved workflow.

Hence, an improved method for compensating the effects of gas or air contamination of acid-base, oxygenation and/or electrolyte status for a venous blood sample taken in a vacuum tube would be advantageous, and in particular a more efficient and/or reliable method would be advantageous.

OBJECT OF THE INVENTION

It is a further object of the present invention to provide an alternative to the prior art.

In particular, it may be seen as an object of the present invention to provide a method that solves the above-mentioned problems of the prior art with gas or air contamination.

SUMMARY OF THE INVENTION

Thus, the above described object and several other objects are intended to be obtained in a first aspect of the invention by providing a computer-implemented method for compensating the effects of gas contamination of acid-base, oxygenation and/or electrolyte status for a venous blood sample, or blood sampled elsewhere, taken in a vacuum tube from a subject, the method comprising:

- providing a blood sample taken in a vacuum tube, said vacuum tube having a known or estimated volume of residual gas ($V_g$) together with the blood sample,

- performing a blood gas measurement of said blood sample resulting in a set of blood variables (BV') indicative of acid-base, oxygenation and/or electrolyte status in said blood sample,

- providing a mathematical model based on a plurality of physiological sub-models simulating development in blood together with said residual gas, the mathematical model comprising:

   o a first sub-model describing the diffusion of gasses between the residual gas and the blood sample,
   o a second sub-model describing the effects of the diffusing gas on the blood in the vacuum tube, and
   o a third sub-model describing the effects of the diffusing gas on the residual gas in the vacuum tube, and

- applying said mathematical model to calculate a modified set of blood variables (BV) indicative of the corresponding acid-base, oxygenation and/or electrolyte status of said blood sample so as to at least partly compensate for the physical and/or chemical interactions between the residual gasses and the blood sample in the time following sampling and before blood gas measurement.

The invention is particularly, but not exclusively, advantageous for obtaining a method to simulate the changes in blood variables seen in a vacuum tube because of gas or air contamination. This is advantageous as the use of vacuum tubes for routine acid-base and oxygen analysis is expected to significantly improve clinical workflows. It is common that blood sampling for acid-base and oxygenation status be taken at different time-points, and by different personnel, than vacuum tube sampling of blood.

By accounting and correcting for the contamination of the blood sample due to residual gas, the present invention may allow an acid-base sample to be drawn with other samples in vacuum tubes, and allow for the calculation of the actual values of blood variables seen in the patient.

The effects of gas distribution between blood and gas phases in the vacuum tube are thought to occur rapidly, for example within 5 min. or 10 min. However, it is contemplated that the present invention can be combined with other mathematical models for compensating a delay between sampling and blood gas measurements accounting for in particular the acid produced due to the red blood cell (RBC) metabolism.

Definitions:

In the context of the present invention, it's to be understood that the method may be used to - at least partly - compensate for the physical and/or chemical interactions between the residual gasses and the blood sample in the time following sampling and before blood gas measurement by for example calculating or simulating to obtain a modified set of blood variables so as to obtain improved knowledge about the blood variable(s) at, or around, the actual time of sampling and thereby mitigate, reduce, indemnify, decrease, reduce, lessen and/or abate the effect of the said contamination, and thereby correspondingly increase the knowledge, in particularly the accuracy, about the blood variable(s) for use in any further medical and/or diagnostic procedure for a subject, such as a human patient in, or near, a clinical environment. Further, when referring to a set of blood variables, it may also as a special case be just one blood variable, which is measured and where the time delay is compensated by the present invention.

In the context of the present invention, it is to be understood that the provided blood sample may be prepared by a health care professional working in a clinic or a hospital, such as a nurse or a doctor, and that the actual taking of the blood sample does not form part of the claimed invention, but the blood sample is nevertheless used as an input sample for further analysis in a dedicated blood gas measurement apparatus or system.

The present invention may be applied for venous blood samples taken in a vacuum tube in, or near, a clinical environment or a hospital. It may be understood that venous blood is collected from a vein, and this is the most common type of blood sample used for a wide range of tests, including metabolic panels and blood cultures. Furthermore, the skilled person in blood sampling will understand that venous blood is sampled for various blood measurements. Typically, for venous blood a puncture is performed using a so-called butterfly needle. This needle may then be connected to a holder, to which multiple tubes (so-called vacutainers or vacuum tubes) can be attached. This means than many venous blood samples can be taken from a single needle puncture. Using butterfly needle puncture, blood samples are typically drawn into vacuum tubes or vacutainers. These tubes contain a partial vacuum such that when connected to the butterfly needle the under-pressure in the tube draws blood from the line. The nature of these tubes, i.e. the partial vacuum, means that following sampling a residual volume of air or gas will be present in the tube. While this does not present a problem for most venous blood measurements, for measurement of acid-base and oxygen status, the presence of air in the sampling tube is problematic. Carbon dioxide will diffuse out of the blood into the air, and oxygen from the air into the blood, changing the blood's status and meaning that accurate measurements of acid-base and oxygen status are not possible or difficult. This problem is well known, and solved by the present invention in a new and advantageous manner.

The skilled person in taking blood sample in vacuum tubes will also understand that the residual air or gas volume when taking a blood sample in a vacuum tube may be measured or estimated, for example from either measuring lines on the vacuum tube or estimated as a fraction of the total volume, which is normally well-known. The volume of residual gas may thus be inputted as a residual volume ($V_g$) value into the mathematical model according to the present invention. It is contemplated that this value of the residual volume may also be set to a standard value, or changed as one value is chosen from a set of standard values.

In the context of the present invention, it is further to be understood that a mathematical model may be an abstract representation of a real-world system using mathematical concepts and language. It may serve to describe, analyze, and predict the behaviour of that system. The process of creating such a model is known as mathematical modelling as the skilled person in modelling will readily understand. In the context of the present invention, it's further to be understood that a physiological model may be a mathematical or computational representation that simulates the biological functions and processes of living organisms or systems. Such physiological models or sub-models may be used to understand, predict, and analyze how various physiological systems operate under different conditions. In particular the present invention applies a mathematical model comprising a plurality of physiological sub-models for describing the effects gas contamination occurring during the time delay between the sampling and measurement of a blood sample. Each physiological sub-model may be developed and tested alone, or in combination with other physiological sub-models to simulate the whole physiological system, or parts thereof. In particular, one or more physiological sub-models may be tested for their impact on the overall mathematical model via sensitivity analysis or other mathematically suitable methods, and if the impact is limited one, or more, sub-model(s) may be omitted in the overall physiological model, or only applied in some situations or circumstances, as it will be explained in more detail below.

The blood variables relevant in the context of the present invention may be conventionally measured by a blood gas measurement apparatus or system as the skilled person in blood measurements will readily understand, such as systems from Radiometer, Roche, Abbot Laboratories, GE Healthcare, Medtronic, Siemens Healthineers, Masimo, Philips Healthcare, Nova Biomedical etc. However, to the best of the inventors' knowledge none of these manufacturers have the ability to compensate or mitigate for any gas contamination from sampling to measurement of the blood sample in a vacuum tube using a mathematical model with various physiological sub-models like the present invention.

In advantageous embodiments, the mathematical model may be based on an assumption that the vacuum tube is completely closed so that the total mass of oxygen and carbon dioxide is constant regardless of gas diffusion between blood and the gas phase in the vacuum tube.

In other advantageous embodiments, the second sub-model describing the effects of the diffusing gas in the blood in the vacuum tube may be calculating total concentrations of $O_2$ and $CO_2$, along with the buffer base concentration in blood, from measurements in the blood and/or gas partial pressures due the diffusion according to, or substantially according to, the equation:

$$tCO_{2,b}, tO_{2,b}, BB_b = acid\ base\ model(\ pHp, PCO_{2,b}, PO_{2,b}, tHb, T_b)$$

wherein the acid base model refers to a biochemical mathematical model of the acid-base chemistry of blood as the skilled person in blood gas measurements will readily understand.

In other valuable embodiments, the mathematical model may take into account a dependency on temperature of the gas phase and/or the blood phase in the vacuum tube during the time delay (TD) from blood sampling to the blood gas measurement. Studies and sensitivity analysis performed by the present inventors have indicated that temperature is important when modelling for the gas contamination. In yet other valuable embodiments, the mathematical model may alternatively or additionally take into account a dependency on temperature of the gas phase and/or the blood phase the during the blood gas measurement. Typically, the blood sample is heated prior and/or during measurement to 37 deg. C.

In preferred embodiments, the third sub-model describing the effects of the diffusing gas on the residual gas in the vacuum tube, the third sub-model may be describing for, one or more, of the following:

- the relationship between partial pressure and fraction of 02 and CO2 depending on atmospheric pressure (AP),
- the volume of $O_2$ and $CO_2$ in the gas phase as the fraction of these gases multiplied by the volume of the gas phase ($V_g$),
- the mass of 02 and CO2 from the volume of these gasses and the molar density ($\rho$) of an ideal gas, and
- the total concentration of 02 and CO2 in the gas phase can then be calculated as mass divided by the volume of the gas phase ($V_g$).

In advantageous embodiments, the first sub-model may be based on an assumption that the partial pressure of oxygen in the gas phase, $PO_{2,g}$, is equal to or mathematically related to the partial pressure of oxygen in the blood phase, $PO_{2,b}$ in the vacuum tube.

In yet other advantageous embodiments, the first sub-model may be based on an assumption that the partial pressure of carbon dioxide in the gas phase, $PCO_{2,g}$, is equal to or mathematically related to the partial pressure of carbon dioxide in the blood phase, $PCO_{2,b}$ in the vacuum tube.

For starting the modelling, the mathematical model may be based on an assumption that immediately after blood sampling, the gas phase in the vacuum tube will have fractions of oxygen and carbon dioxide being approximately equal to atmospheric conditions.

In some advantageous embodiments, the mathematical model may take into account that there is not complete equilibrium between the partial pressures of carbon dioxide or oxygen in the gas phase, ($PCO_{2,g}$, $PO_{2,g}$) with the partial pressure of carbon oxygen or oxygen in the blood phase, ($PCO_{2,b}$, $PO_{2,b}$) in the vacuum tube, at least during the initial part of the time delay (TD) from blood sampling to the blood gas measurement.

In a preferred embodiment, the mathematical model may calculate backwards in time from blood variables (BV') in the measured blood to blood gas variables (BV) in the sampled blood before diffusion has occurred. This is highly advantageous because the present invention may be applied for obtaining more accurate blood variables where the effect of the gas contamination for venous blood samples taken by a vacuum tube is at least partly compensated for or mitigated.

In another preferred embodiment, the mathematical model may calculate forwards in time from blood variables (BV) in the sample blood to blood variables (BV') when measured after diffusion has occurred. Thus, the present invention may also be applied for example for testing how a blood sample changes due to gas contamination, which may be relevant for quality control and documentation in laboratories working for clinics or hospitals.

In yet other advantageous embodiments, the set of blood variables (BV) may be chosen from the group consisting of: pH, $PCO_2$, $POz$, $SO_2$, Hb, FMetHb, FCOHb, glucose, lactate, $Na^+$, $Cl^-$, $Ca^{2+}$, and/or $K^+$, and other blood variables readily available for the skilled person in blood gas measurements. It is of course to be understood that the mentioning of chemical species, such as glucose, lactate, $Na^+$, $Cl^-$, or $Ca^{2+}$, etc. in the context of blood gas measurements are normally meant as concentration of these species, e.g. mmol/litre or other suitable chemical units such as charge equivalents e.g. meq/l, often indicated in chemistry by square brackets, i.e. $[Na^+]$, or $[Cl^-]$, as the skilled person in biochemistry, in particular blood gas measurements, will immediately understand. It should also be noted that "P" refers to partial pressure, "S" to saturation and "F" to fraction in the general context of the present application as familiar for the skilled person in biochemistry, in particular blood gas measurements.

In yet other valuable embodiments, the plurality of sub-models may be simplified, or their number reduced, in the sense that only a limited number of the physiological equations and elements are implemented as compared to the full extended model presented in the detailed description of an embodiment below. Thus, based on the insights and experience from testing and/or modelling of the present invention a number of situations may readily be identified for such reduced and simplified modelling, in particular if only a relatively small number of blood variables are of interest for compensating the effect of gas contamination.

In a second aspect, the invention relates to a computer-implemented system for compensating the effects of gas contamination of acid-base, oxygenation and/or electrolyte status for a venous blood sample, or blood sampled elsewhere, taken in a vacuum tube from an associated subject, the system being arranged for:

- receiving a blood sample taken in a vacuum tube, said vacuum tube having a known or estimated volume of residual gas ($V_g$) together with the blood sample,

- performing a blood gas measurement of said blood sample resulting in a set of blood variables (BV') indicative of acid-base, oxygenation and/or electrolyte status in said blood sample,

- operating a mathematical model based on a plurality of physiological sub-models simulating development in blood together with said residual gas, the mathematical model comprising:

    o a first sub-model describing the diffusion of gasses between the residual gas and the blood sample,
    o a second sub-model describing the effects of the diffusing gas on the blood in the vacuum tube, and
    o a third sub-model describing the effects of the diffusing gas on the residual gas in the vacuum tube, and

- applying said mathematical model to calculate a modified set of blood variables (BV) indicative of the corresponding acid-base, oxygenation and/or electrolyte status of said blood sample so as to at least partly compensate for the physical and/or chemical interactions between the residual gasses and the blood sample in the time following sampling and before blood gas measurement.

In a third aspect, the invention relates to a computer program product being adapted to enable a computer system comprising of at least one computer having data storage means in connection therewith to control a system according to the second aspect of the invention, such as a computer program product comprising instructions which, when the program is executed by a computer, cause the computer to carry out the steps of the method of first aspect of the invention.

This aspect of the invention is particularly, but not exclusively, advantageous in that the present invention may be accomplished by a computer program product enabling a computer system to carry out the operations of the apparatus/system of the second aspect of the invention when down- or uploaded into the computer system. Such a computer program product may be provided on any kind of computer readable medium, or through a network.

In a fourth aspect, the invention relates to the use of the method and/or the system for compensating the effects of gas contamination of acid-base, oxygenation and/or electrolyte status for a venous blood sample, or blood sampled elsewhere, taken in a vacuum tube from a subject.

In a preferred embodiment, preferably based on a human sample or veterinary sample, the subject is a mammal, preferably a human, preferably use wherein the subject is a healthy subject or a subject with a disease, such as respiratory diseases, cardiovascular diseases or metabolic diseases, causing low oxygen levels, abnormal carbon dioxide levels, and/or electrolyte abnormalities.

The use wherein the location of obtaining the sample is at a different location than the location of estimating or measuring the blood variable(s), such as in different buildings or rooms.

Use of a method according to the first aspect or a system according to the second aspect, wherein the subject is a mammal, preferably a human. In an embodiment, the mammal is selected from the group consisting of a pet, such as a dog or cat, a racing animal such as a horse, dog or camel, or a farm animal, such as a cow, goat, camel, or horse.

The use wherein the subject is a healthy subject or a subject with a disease, such as respiratory diseases, cardiovascular diseases or metabolic diseases, causing low oxygen levels, abnormal carbon dioxide levels, and/or electrolyte abnormalities.

In an embodiment, the disease is selected from the group consisting of:
Chronic Obstructive Pulmonary Disease (COPD), asthma, pneumonia, pulmonary embolism, pulmonary fibrosis, congestive heart failure, sleep apnea, anemia, Acute Respiratory Distress Syndrome (ARDS), and COVID-19, but not limited to any of these specific diseases.

The individual aspects of the present invention may each be combined with any of the other aspects. These and other aspects of the invention will be apparent from the following description with reference to the described embodiments.

## BRIEF DESCRIPTION OF THE FIGURES

The invention will now be described in more detail with regard to the accompanying figures. The figures show one way of implementing the present invention and is not to be construed as being limiting to other possible embodiments falling within the scope of the attached claim set.

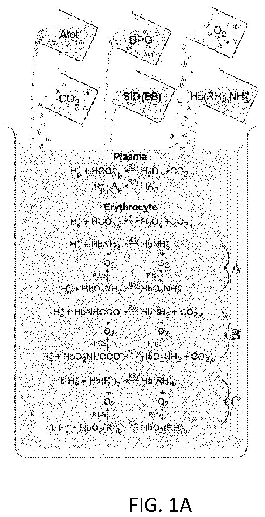

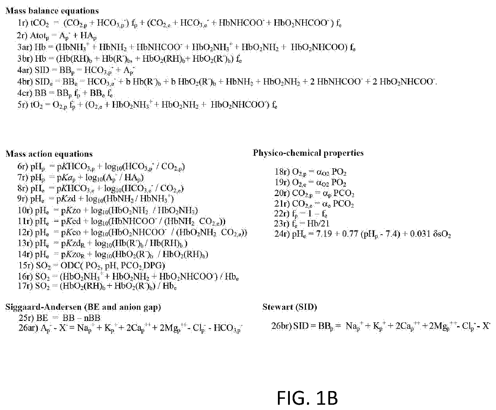

Figures 1A and 1B show an overview of the mathematical model of acid-base chemistry of the blood, the model includes chemical reactions describing bicarbonate and non-bicarbonate buffering in plasma and erythrocyte fractions, including the competitive binding of $H^+$, $O_2$ and $CO_2$ to hemoglobin, and equations describing the mass balance of state variables (1-5r), mass action equations describing chemical reactions (6r-17r) and physiochemical properties including gas solubility (18r to 21r), blood fractions (22r, 23r) and pH differences across the RBC membrane (24r), and the model includes calculation of the base excess (BE) (25r), and equations representing two different methods to account for electrical neutrality in the blood (26ar, 26br),

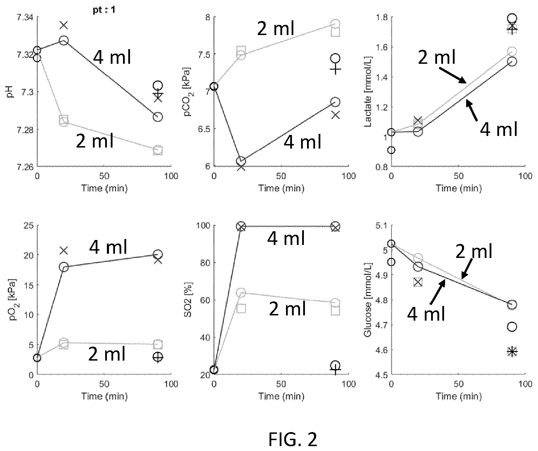

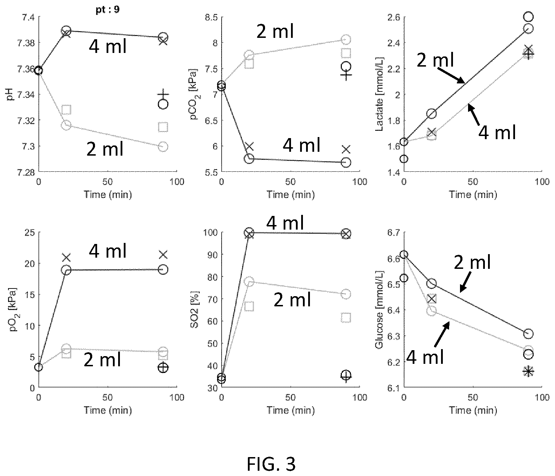

Figures 2 and 3 show two representative patients (number 1 & 9) with measurements (circles) and model simulations (plus signs, crosses, squares), for standard blood gas analysis syringes (circles, plus-signs), 2 ml vacuum tubes (circles, squares) and 4 ml vacuum tubes (circles, crosses), and lines connecting measurements from baseline, measured in standard blood gas analysis syringes without gas contamination, to 20 and 90 minute values for 2ml vacuum tubes and 4 ml vacuum tubes, and

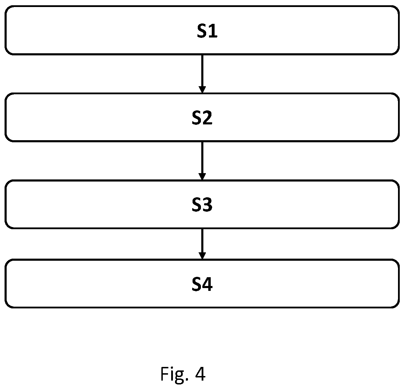

Figure 4 is a schematic system-chart representing an out-line of/in detail the operations of the method and/or the computer program product according to the invention.

## DETAILED DESCRIPTION OF AN EMBODIMENT

**Introduction**

The following text describes a detailed embodiment of the invention including description of the mathematical equations necessary to perform the calculations. In addition, it describes the application of this embodiment in two patient cases, the data of which is illustrated in Figures 2 and 3. These data are drawn from a clinical study in 12 normal subjects and exemplify use of the method. For each subject blood samples have been drawn into three different sample tubes at the same point in time. One of these sample tubes was standard blood gas analysis syringes, for which two samples were taken and one measured at time zero and the other at 90 minutes. The other two sample tubes were vacuum tubes drawing 2ml or 4 ml of blood respectively with gas and blood volumes measured. Two each of these were sampled at time zero and analysed at 20 and 90 minutes, respectively. By evaluating these samples at 20 minutes and 90 minutes following sampling the effects of gas transport between the blood and gas phases of the vacuum tube, as well as the effects of the delay in sampling, were studied. All samples were stored at room temperature prior to analysis. In each sample, values of the following variables were measured: pH, $pCO_2$, $pO_2$, $SO_2$, Hb, FMetHb, FCOHb, glucose and lactate.

The mathematical equations which account for the diffusion of oxygen and carbon dioxide between gas and blood phases and the resulting effects on these phases are described below. These equations can be used to calculate the effects of the gas diffusion between phases both forwards in time from sampling to analysis, as illustrated in Figures 2 and 3 and backwards in time, calculating back to the initial conditions in the blood prior to contamination.

The principle of the method is that in a closed vacuum tube containing blood and residual air, the total mass of oxygen and carbon dioxide is constant regardless of gas diffusion between blood and gas phases. It is therefore possible to calculate the total mass of $O_2$ and $CO_2$ in the blood and gas phases together, and then simulate the results of partial or complete diffusion equilibrium between the phases. This allows calculation of all acid-base and oxygen variables in the blood before or after gas diffusion.

To account for concentration of $O_2$ and $CO_2$ in the gas phase, equations are required linking mass and partial pressure in the gas phase, equations 1-9 as follows.

Equations 1 and 2 describe the relationship between partial pressure and fraction of $O_2$ and $CO_2$ depending on atmospheric pressure (*AP*).

$$PO_{2,g} = FO_{2,g} * AP \quad (1)$$

$$PCO_{2,g} = FCO_{2,g} * AP \quad (2)$$

Equations 3 and 4 account for the volume of $O_2$ and $CO_2$ in the gas phase as the fraction of these gases multiplied by the volume of the gas phase ($V_g$).

$$VO_{2,g} = FO_{2,g} * V_g \quad (3)$$

$$VCO_{2,g} = FCO_{2,g} * V_g \quad (4)$$

Equations 5 and 6 account for the mass of $O_2$ and $CO_2$ from the volume of these gasses and the molar density ($\rho$) of an ideal gas, as

$$mO_{2,g} = VO_{2,g} * \rho \quad (5)$$

$$mCO_{2,g} = VCO_{2,g} * \rho \quad (6)$$

where the molar density is calculated as in equation 7, using the gas constant ($R$) and gas temperature ($T_g$).

$$\rho = AP / R * T_g \quad (7)$$

The total concentration of $O_2$ and $CO_2$ in the gas phase can then be calculated as mass divided by the volume of the gas phase ($V_g$), equations 8 and 9.

$$tO_{2,g} = mO_{2,g} / V_g \quad (8)$$

$$tCO_{2,g} = mCO_{2,g} / V_g \quad (9)$$

To account for concentration of $O_2$ and $CO_2$ in the blood phase and link this to the partial pressure driving diffusion between gas and blood, equations are required linking mass and partial pressure in the blood. The equations describing this relationship have been previously described in the mathematical model of blood acid-base chemistry of Rees and Andreassen, cf. Rees SE, Andreassen S. Mathematical Models of Oxygen and Carbon Dioxide Storage and Transport: The Acid-Base Chemistry of Blood. Crit Rev Biomed Eng. 2005;33(3):209-64. This model, illustrated in Figure 1A and 1B, describes the plasma and red blood cell buffering including mass conservation equations, mass action/reaction equations, physiochemical properties, an empirical relationship for the link between plasma and erythrocyte pH based on the Donnan effect and description of plasma and erythrocyte fractions from hemoglobin concentration. The model includes state variables describing: total oxygen concentration ($tO_{2b}$), total carbon dioxide concentration ($tCO_{2,b}$), total hemoglobin concentration (tHb), total buffer base concentration ($BB_b$), and total plasma non-bicarbonate buffer concentration ($Atot_p$), the standard value for which in normal blood is used in calculations performed here, but which may be modified to other values if values of plasma total protein or albumin concentration are known. Values of state variables can be calculated from clinical measurements in a blood gas sample, and simulations performed for addition or removal of acid or $CO_2$; or equilibration of blood at $CO_2$ or $O_2$ partial pressures. The total concentrations are related to values of acid-base and oxygen status according to equation 10a where the acid-base model is the implementation of the mode of Rees and Andreassen illustrated in figures 1A and 1B.

In the implementation used to analyse these data, equation 10a is modified to account for an addition of acid due to red blood cell metabolism on the storage of blood ($\delta acid$). A value of 0.008 meq/l/min is applied in all subjects studied here with this value reducing the buffer base concentration of the blood ($BB_b$). As such equation 10a is implemented in a modified form illustrated in 10b. The net effects of such acid addition are to decrease pH and increase $PCO_2$ levels.

$$tCO_{2,b}, tO_{2,b}, BB_b = acid\ base\ model(\ pHp, PCO_{2,b}, PO_{2,b}, tHb, T_b) \quad (10a)$$

$$tCO_{2,b}, tO_{2,b}, \ pH_p = acid \ base \ model( \ BB_b - \delta acid, PCO_{2,b}, PO_{2,b}, \text{tHb}, T_b) \qquad (10b)$$

As described previously, when $O_2$ and $CO_2$ diffuse between gas and blood phases, the total concentration of these gases in the tube, as a whole, is assumed to remain constant. Vacuum tubes are often stored for long durations without loss of under-pressure, and it is therefore likely that little gas diffusion occurs across the wall of the tube. The total concentration of each gas in the tube as a whole is accounted for by the sum of those from the gas and blood phases, weighted by the fraction of each phase as described in equations 11 and 12.

$$tO_{2,tube} = tO_{2,b} * f_b \ + \ tO_{2,g} \ f_g \qquad (11)$$

$$tCO_{2,tube} = tCO_{2,b} * f_b \ + \ tCO_{2,g} \ f_g \qquad (12)$$

Diffusion of $O_2$ and $CO_2$ between gas and blood phases is described by equations 13 and 14. Following blood sampling diffusion of $O_2$ will proceed from the gas into blood and $CO_2$ from blood to gas. At equilibrium, some time later, partial pressures in gas and blood will be equivalent, but it is unknown whether such equilibrium occurs over relatively short durations. Equations 13 and 14 therefore describe the partial pressure relationships with parameters used to account for the fraction of equilibration between blood and gas phases.

$$PO_{2,g} = f_{equib,O_2} * \ PO_{2,b} \qquad (13)$$

$$PCO_{2,g} = f_{equib,CO_2} * \ PCO_{2,b} \qquad (14)$$

The mathematical model presented in the embodiment here includes 14 equations, plus those included in Figure 1A and 1B. To simulate either forward in time from sampling to measurement or backward in time from measurement to sampling requiring values measured in the blood either at the sample or measurement time, values of the model parameters describing the fraction of equilibrium ($f_{equib,O_2}, f_{equib,CO_2}$) the measurements or approximations of the volume of gas ($V_g$) and blood ($V_b$) in the phases, and the temperature of blood ($T_b$) and gas ($T_g$). All 14 equations can then be solved to calculate all variables either forward in time to the measurement values or backward in time to the values in the original sample. In the 12 subjects use to validate this method parameter $f_{equib,O_2} = 1$, $f_{equib,CO_2} = 0.36$ for 4 ml tubes and , $f_{equib,CO_2} = 0.63$ for 2 ml tubes, $T_g = 22$ degrees centigrade, i.e. room temperature, and $T_b = 37$ degrees centigrade for sampled blood or for that measured in the blood gas analyser, or $T_b = 22$ degrees centigrade following equilibrium with gas in the vacuum tube. Gas in the tube on blood sampling was assumed to be air at normal atmospheric pressure. A production of acid due to red blood cell metabolism $\delta acid = 0.008$ meq/l/min is also assumed.

Figures 2 and 3 illustrate data sets and model simulations for two subjects for 2ml and 4 ml vacuum tubes, and standard blood gas analysis syringes. These data and simulations are typical of all those for all subjects. For the 2 ml vacuum tube, $PCO_2$ values fall dramatically after 20 minutes. This is likely due to $CO_2$ transport from blood to the gas phase. For the 4ml tube $PCO_2$ values are increased after 20 minutes, which is due to the red blood cell metabolism production of strong acid and hence dissociation of bicarbonate in the plasma. For the 4ml vacuum tube the size of the gas fraction is relatively small and hence the diffusion of $CO_2$ out of the blood is small. Increases in $PCO_2$ due to acid production therefore outweigh decreases due to diffusion. For 90 minutes further changes in pH and $PCO_2$ above the 20-minute level are likely to be dominated by acid-production, with the majority of diffusion occurring over a relatively short time. This is consistent with increasing $PCO_2$ levels and decreasing pH levels from 20 to 90 minutes. For pH, $PCO_2$, POz and SO2 model simulations accurately predict measurements. In addition, as increases in lactate concentration are known to be equivalent to acid production, and decreases in glucose equivalent to half of acid production, accurate simulation of changes in lactate and glucose concentrations are also possible as also shown on Figures 2 and 3.

Figure 4 is a schematic system-chart representing an out-line of/in detail the operations of the method and/or the computer program product according to the invention i.e. a computer-implemented method for compensating the effects of gas contamination of acid-base, oxygenation and/or electrolyte status for a venous blood sample, or blood sampled elsewhere, taken in a vacuum tube from a subject, the method comprising:

**S1** providing a blood sample taken in a vacuum tube, said vacuum tube having a known or estimated volume of residual gas ($V_g$) together with the blood sample, such as a venous blood sample obtained in vacuum tube from a patient in a clinic or a hospital,

**S2** performing a blood gas measurement of said blood sample resulting in a set of blood variables (BV') indicative of acid-base, oxygenation and/or electrolyte status in said blood sample,

**S3** providing a mathematical model, such as the model shown in Figure 1, based on a plurality of physiological sub-models simulating development in blood together with said residual gas, the mathematical model comprising:

o a first sub-model describing the diffusion of gasses between the residual gas and the blood sample,
o a second sub-model describing the effects of the diffusing gas on the blood in the vacuum tube, and
o a third sub-model describing the effects of the diffusing gas on the residual gas in the vacuum tube, and

**S4** applying said mathematical model to calculate a modified set of blood variables (BV) indicative of the corresponding acid-base, oxygenation and/or electrolyte status of said blood sample so as to at least partly compensate for the physical and/or chemical interactions between the residual gasses and the blood sample in the time following sampling and before blood gas measurement, cf. Figures 2 and 4 for simulations of some blood variables for two patients taking into account the gas contamination.

In short, the invention relates to a computer-implemented method for compensating the effects of gas contamination of acid-base, oxygenation and/or electrolyte status for a venous blood sample, or blood sampled elsewhere, taken in a vacuum tube from a subject, such as human patient. The vacuum tube has a volume of residual gas ($V_g$) together with the blood. The method has a mathematical model with physiological sub-models simulating development in blood together with this residual gas, including sub-models describing: 1) the diffusion of gasses between the residual gas and the blood sample, 2) the effects of the diffusing gas on the blood in the vacuum tube, and 3) the effects of the diffusing gas on the residual gas in the vacuum tube. The mathematical model then calculates a modified set of blood variables (BV) to compensate for the physical and/or chemical interactions between the residual gasses and the blood sample in the time following sampling and before blood gas measurement. By accounting for this contamination of the blood sample, and hence calculation of the actual values of blood variables seen in the patient is possible, cf. Figures 2 and 3.

The invention can be implemented by means of hardware, software, firmware or any combination of these. The invention or some of the features thereof can also be implemented as software running on one or more data processors and/or digital signal processors.

The individual elements of an embodiment of the invention may be physically, functionally and logically implemented in any suitable way such as in a single unit, in a plurality of units or as part of separate functional units. The invention may be implemented in a single unit, or be both physically and functionally distributed between different units and processors.

Although the present invention has been described in connection with the specified embodiments, it should not be construed as being in any way limited to the presented examples. The scope of the present invention is to be interpreted in the light of the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

**Claims**

1. A computer-implemented method for compensating the effects of gas contamination of acid-base, oxygenation and/or electrolyte status for a venous blood sample, or blood sampled elsewhere, taken in a vacuum tube from a subject, the method comprising:

- providing a blood sample taken in a vacuum tube, said vacuum tube having a known or estimated volume of residual gas ($V_g$) together with the blood sample,
- performing a blood gas measurement of said blood sample resulting in a set of blood variables (BV') indicative of acid-base, oxygenation and/or electrolyte status in said blood sample,
- providing a mathematical model based on a plurality of physiological sub-models simulating development in blood together with said residual gas, the mathematical model comprising:

o a first sub-model describing the diffusion of gasses between the residual gas and the blood sample,
o a second sub-model describing the effects of the diffusing gas on the blood in the vacuum tube, and
o a third sub-model describing the effects of the diffusing gas on the residual gas in the vacuum tube, and

- applying said mathematical model to calculate a modified set of blood variables (BV) indicative of the corresponding acid-base, oxygenation and/or electrolyte status of said blood sample so as to at least partly compensate for the physical and/or chemical interactions between the residual gasses and the blood sample in the time following sampling and before blood gas measurement.

2. The computer-implemented method according to claim 1, wherein the mathematical model is based on an assumption that the vacuum tube is completely closed so that the total mass of oxygen and carbon dioxide is constant regardless of gas diffusion between blood and the gas phase in the vacuum tube.

3. The computer-implemented method according to any of the preceding claims, wherein the second sub-model describing the effects of the diffusing gas on the blood in the vacuum tube is calculating total concentrations of $O_2$ and $CO_2$, along with the buffer base concentration in blood, from measurements in the blood and/or gas partial pressures due the diffusion according to, or substantially according to, the equation:

$$tCO_{2,b}, tO_{2,b}, BB_b = acid\ base\ model(\,pHp, PCO_{2,b}, PO_{2,b}, tHb, T_b),$$

wherein the acid base model refers to a biochemical mathematical model of the acid-base chemistry of blood.

4. The computer-implemented method according to any of the preceding claims, wherein the mathematical model takes into account a dependency on temperature of the gas phase and/or the blood phase in the vacuum tube during the time delay (TD) from blood sampling to the blood gas measurement.

5. The computer-implemented method according to any of the preceding claims, wherein the mathematical model takes into account a dependency on temperature of the gas phase and/or the blood phase the during the blood gas measurement.

6. The computer-implemented method according to any of the preceding claims, wherein the third sub-model describing the effects of the diffusing gas on the residual gas in the vacuum tube, the third sub-model describing for, one or more, of the following:

   - the relationship between partial pressure and fraction of $O_2$ and $CO_2$ depending on atmospheric pressure (AP),
   - the volume of $O_2$ and $CO_2$ in the gas phase as the fraction of these gases multiplied by the volume of the gas phase ($V_g$),
   - the mass of $O_2$ and $CO_2$ from the volume of these gasses and the molar density ($\rho$) of an ideal gas, and
   - the total concentration of $O_2$ and $CO_2$ in the gas phase can then be calculated as mass divided by the volume of the gas phase ($V_g$).

7. The computer-implemented method according to any of the preceding claims, wherein the first sub-model is based on an assumption that the partial pressure of oxygen in the gas phase, $PO_{2,g}$, is equal to or mathematically related to the partial pressure of oxygen in the blood phase, $PO_{2,b}$ in the vacuum tube.

8. The computer-implemented method according to any of the preceding claims, wherein the first sub-model is based on an assumption that the partial pressure of carbon dioxide in the gas phase, $PCO_{2,g}$, is equal to or mathematically related to the partial pressure of carbon dioxide in the blood phase, $PCO_{2,b}$ in the vacuum tube.

9. The computer-implemented method according to any of the preceding claims, wherein the mathematical model is based on an assumption that immediately after blood sampling, the gas phase in the vacuum tube will have fractions of oxygen and carbon dioxide being approximately equal to atmospheric conditions.

10. The computer-implemented method according to any of the preceding claims, wherein the mathematical model takes into account that the partial pressure of carbon dioxide or oxygen in the gas phase, ($PCO_{2,g}$, $PCO_{2,g}$) is not in equilibrium with the partial pressure of carbon oxygen or oxygen in the blood phase, ($PCO_{2,b}$, $PCO_{2,b}$) in the vacuum tube, at least during the initial part of the time delay (TD) from blood sampling to the blood gas measurement.

11. The computer-implemented method according to any of the preceding claims, wherein the mathematical model calculates backwards in time from blood variables (BV') in the measured blood to blood gas variables (BV) in the sampled blood before diffusion has occurred.

12. The computer-implemented method according to any of the preceding claims, wherein the mathematical model calculates forwards in time from blood variables (BV) in the sample blood to blood variables (BV') when measured after diffusion has occurred.

13. The computer-implemented method according to any of the preceding claims, wherein the set of blood variables (BV) are chosen from the group consisting of: pH, $PCO_2$, POz, $SO_2$, Hb, FMetHb, FCOHb, glucose, lactate, $Na^+$, $Cl^-$, $Ca^{2+}$, and/or $K^+$.

14. A computer-implemented system for compensating the effects of gas contamination of acid-base, oxygenation and/or electrolyte status for a venous blood sample, or blood sampled elsewhere, taken in a vacuum tube from an associated subject, the system being arranged for:

- receiving a blood sample taken in a vacuum tube, said vacuum tube having a known or estimated volume of residual gas ($V_g$) together with the blood sample,
- performing a blood gas measurement of said blood sample resulting in a set of blood variables (BV') indicative of acid-base, oxygenation and/or electrolyte status in said blood sample,
- operating a mathematical model based on a plurality of physiological sub-models simulating development in blood together with said residual gas, the mathematical model comprising:

o a first sub-model describing the diffusion of gasses between the residual gas and the blood sample,
o a second sub-model describing the effects of the diffusing gas on the blood in the vacuum tube, and
o a third sub-model describing the effects of the diffusing gas on the residual gas in the vacuum tube, and

- applying said mathematical model to calculate a modified set of blood variables (BV) indicative of the corresponding acid-base, oxygenation and/or electrolyte status of said blood sample so as to at least partly compensate for the physical and/or chemical interactions between the residual gasses and the blood sample in the time following sampling and before blood gas measurement.

15. A computer program product being adapted to enable a computer system comprising at least one computer having data storage means in connection therewith to control a system according to claim 14, such as a computer program product comprising instructions which, when the program is executed by a computer, cause the computer to carry out the steps of the method of claim 1.

16. The use of the method and/or the system according to any of claims 1-15 for compensating the effects of gas contamination of acid-base, oxygenation and/or electrolyte status for a venous blood sample, or blood sampled elsewhere, taken in a vacuum tube from a subject.

FIG. 1A

**Mass balance equations**

1r) $tCO_2 = (CO_{2,p} + HCO_{3,p}^-) f_p + (CO_{2,e} + HCO_{3,e}^- + HbNHCOO^- + HbO_2NHCOO^-) f_e$

2r) $Atot_p = A_p^- + HA_p$

3ar) $Hb = (HbNH_3^+ + HbNH_2 + HbNHCOO^- + HbO_2NH_3^+ + HbO_2NH_2 + HbO_2NHCOO) f_e$

3br) $Hb = (Hb(RH)_b + Hb(R^-)_b + HbO_2(RH)_b + HbO_2(R^-)_b) f_e$

4ar) $SID = BB_p = HCO_{3,p}^- + A_p^-$

4br) $SID_e = BB_e = HCO_{3,e}^- + b\ Hb(R^-)_b + b\ HbO_2(R^-)_b + HbNH_2 + HbO_2NH_2 + 2\ HbNHCOO^- + 2\ HbO_2NHCOO^-$

4cr) $BB = BB_p f_p + BB_e f_e$

5r) $tO_2 = O_{2,p} f_p + (O_{2,e} + HbO_2NH_3^+ + HbO_2NH_2 + HbO_2NHCOO^-) f_e$

**Mass action equations**

6r) $pH_p = pKHCO_{3,p} + log_{10}(HCO_{3,p}^- / CO_{2,p})$

7r) $pH_p = pKa_p + log_{10}(A_p^- / HA_p)$

8r) $pH_e = pKHCO_{3,e} + log_{10}(HCO_{3,e}^- / CO_{2,e})$

9r) $pH_e = pKzd + log_{10}(HbNH_2 / HbNH_3^+)$

10r) $pH_e = pKzo + log_{10}(HbO_2NH_2 / HbO_2NH_3)$

11r) $pH_e = pKcd + log_{10}(HbNHCOO^- / (HbNH_2\ CO_{2,e}))$

12r) $pH_e = pKco + log_{10}(HbO_2NHCOO^- / (HbO_2NH_2\ CO_{2,e}))$

13r) $pH_e = pKzd_R + log_{10}(Hb(R^-)_b / Hb(RH)_b)$

14r) $pH_e = pKzo_R + log_{10}(HbO_2(R^-)_b / HbO_2(RH)_b)$

15r) $SO_2 = ODC(PO_2, pH, PCO_2, DPG)$

16r) $SO_2 = (HbO_2NH_3^+ + HbO_2NH_2 + HbO_2NHCOO^-) / Hb_e$

17r) $SO_2 = (HbO_2(RH)_b + HbO_2(R^-)_b) / Hb_e$

**Physico-chemical properties**

18r) $O_{2,p} = \alpha_{O2}\ PO_2$

19r) $O_{2,e} = \alpha_{O2}\ PO_2$

20r) $CO_{2,p} = \alpha_p\ PCO_2$

21r) $CO_{2,e} = \alpha_e\ PCO_2$

22r) $f_p = 1 - f_e$

23r) $f_e = Hb/21$

24r) $pH_e = 7.19 + 0.77 (pH_p - 7.4) + 0.031\ \delta sO_2$

**Siggaard-Andersen (BE and anion gap)**

25r) $BE = BB - nBB$

26ar) $A_p^- - X^- = Na_p^+ + K_p^+ + 2Ca_p^{++} + 2Mg_p^{++} - Cl_p^- - HCO_{3,p}^-$

**Stewart (SID)**

26br) $SID = BB_p = Na_p^+ + K_p^+ + 2Ca_p^{++} + 2Mg_p^{++} - Cl_p^- - X^-$

FIG. 1B

FIG. 2

EP 4 742 253 A1

FIG. 3

EP 4 742 253 A1

```
┌─────────────────────────────────────┐
│                 S1                   │
└─────────────────────────────────────┘
                    │
                    ▼
┌─────────────────────────────────────┐
│                 S2                   │
└─────────────────────────────────────┘
                    │
                    ▼
┌─────────────────────────────────────┐
│                 S3                   │
└─────────────────────────────────────┘
                    │
                    ▼
┌─────────────────────────────────────┐
│                 S4                   │
└─────────────────────────────────────┘
```

Fig. 4

## EUROPEAN SEARCH REPORT

**Application Number**

EP 24 21 2149

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TANG NGA-YEUNG ET AL: "A comparison of venous blood gas analysis with paired specimens collected in syringes and evacuated blood collection tubes", CLINICA CHIMICA ACTA, ELSEVIER BV, AMSTERDAM, NL, vol. 510, 18 August 2020 (2020-08-18), pages 671-674, XP086284927, ISSN: 0009-8981, DOI: 10.1016/J.CCA.2020.08.023 [retrieved on 2020-08-18] * title, abstract, Materials and Methods, p. 672, 673 and table I * | 1-16 | INV. G16C60/00 G01N33/49 G16B5/30 |
| A | S.E.REES: "Mathematical Models of Oxygen and Carbon Dioxide Storage and Transport: The Acid-Base Chemistry of Blood", CRITICAL REVIEWS IN BIOMEDICAL ENGINEERING., vol. 33, no. 3, 1 January 2005 (2005-01-01), pages 209-264, XP093267397, US ISSN: 0278-940X, DOI: 10.1615/critrevbiomedeng.v33.i3.10 * the whole document * | 1-6 | |
| X,P | NEVIRIAN BAHAREH ET AL: "A physio-chemical mathematical model of the effects of O2 and CO2 diffusion between the blood and gas phases of venous blood sampled in vacuum tubes", CLINICA CHIMICA ACTA, vol. 572, 1 May 2025 (2025-05-01), page 120270, XP093267464, AMSTERDAM, NL ISSN: 0009-8981, DOI: 10.1016/j.cca.2025.120270 * the whole document * | 1 | |

**TECHNICAL FIELDS SEARCHED (IPC)**

G16C
G01N
G16B

The present search report has been drawn up for all claims

| Place of search | Date of completion of the search | Examiner |
|---|---|---|
| Munich | 9 April 2025 | Lüdemann, Susanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P4C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **REES SE** ; **ANDREASSEN S.** Mathematical Models of Oxygen and Carbon Dioxide Storage and Transport: The Acid-Base Chemistry of Blood. *Crit Rev Biomed Eng.*, 2005, vol. 33 (3), 209-64